Europäisches Patentamt

European Patent Office

Office européen des brevets

(11) Publication number: **0 411 974 A1**

# EUROPEAN PATENT APPLICATION

(21) Application number: **90401835.5**

(22) Date of filing: **26.06.90**

(51) Int. Cl.⁵: **C12P 21/08, G01N 33/577,** //C12N15/12,C12Q1/68

The microorganism(s) has (have) been deposited with Intitute Pasteur under number(s) I-883 and I-884.

The title of the invention has been amended (Guidelines for Examination in the EPO, A-III, 7.3).

(30) Priority: **05.07.89 EP 89401931**

(43) Date of publication of application:
**06.02.91 Bulletin 91/06**

(84) Designated Contracting States:
**AT BE CH DE DK ES FR GB GR IT LI LU NL SE**

(71) Applicant: **N.V. INNOGENETICS S.A.**
**Industriepark Zwijnaarde 7, Box 4**
**B-9710 Gent(BE)**

(72) Inventor: **Gheuens, Jan**
**Cottagelaan 43**
**B-8458 Oostduinkerke(BE)**
Inventor: **Van Heuverswyn, Hugo**
**Colmanstraat 62**
**B-9288 Laarne(BE)**

(74) Representative: **Grosset-Fournier, Chantal Catherine et al**
**ERNEST GUTMANN-YVES PLASSERAUD S.A.,**
**67 boulevard Haussmann**
**F-75008 Paris(FR)**

(54) **Monoclonal antibodies to a neurofibrillary tangle antigen.**

(57) The invention relates to a monoclonal antibody characterized by the combination of the following properties :
- it forms an immunological complex with a non-phosphorylated structural epitope of an antigen released from a sonicated NFT preparation, which itself is isolated from the cerebral cortex obtained from a patient having Alzheimer's disease,
- it does not form an immunologically complex with the following proteins or peptides :
. neurofilament proteins
. tau protein
. MAP 2 protein
. ubiquitin
. 4-amyloid peptide,
said monoclonal antibody can be used for the diagnosis in vitro of brain diseases involving neurofibrillary degeneration.

EP 0 411 974 A1

# MONOCLONAL ANTIBODIES INVOLVED IN ALZHEIMER'S DISEASE, HYBRIDOMAS SECRETING THESE MONOCLONAL ANTIBODIES, ANTIGEN RECOGNIZED BY THESE ANTIBODIES, AND THEIR APPLICATION

The invention is relative to new monoclonal antibodies involved in Alzheimer's disease, and to hybridomas secreting such monoclonal antibodies. It is also relative to an antigen which forms an immunological complex with one of said monoclonal antibodies.

The invention also relates to a process for the diagnosis in vitro of Alzheimer's disease.

The most prominent neuropathological abnormalities in the brain, presented in Alzheimer's disease (ADS) comprise cortical atrophy, neuronal loss, senile plaques, neurofibrillary tangles (NFT) and variable amounts of vascular amyloid deposits (Selkoe DJ. Altered structural proteins in plaques and tangles : what do they tell us about the biology of Alzheimer's disease. Neurobiol. Aging 1986; 7: 425-432). Although such NFT can also be found in far lesser numbers in intellectually normal old people, it is their widespread occurence and high number which is typical for Alzheimer's disease. Ultrastructurally, NFT and the dystrophic neurites of senile plaques comprise helical filaments of 10 nm diameter, termed paired helical filaments (PHF), as well as 12-15 nm straight filaments and amorphous elements (Hirano A. Zimmerman HM. Alzheimer neurofibrillary changes. A topographical study. Arch. Neurol. 1962; 7: 227-242 - Price DL, Whitehouse PJ, Struble RG. Cellular pathology in Alzheimer's and Parkinson's disease. Trends Neurosci. 1986; :29-33). In contrast, the central amyloid core of the senile plaques and the vascular amyloid deposits consist of straight 4-8 nm wide filaments. Other abnormal neuronal features found in Alzheimer's disease are granulovacuolar degeneration and Hirano bodies (Price DL, see above mentioned reference).

NFT are also found in a variety of other conditions, e.g. post-encephalitic Parkinsonism, Parkinson-dementia complex of Guam, and subacute sclerosing panencephalitis (Iqbal K, Grundke-Iqbal I, Wisniewski HM. Alterations of the neuronal cytoskeleton in Alzheimer's disease and related conditions. In:Perry G (Ed) Alterations in the Neuronal Cytoskeleton in Alzheimer's disease. Plenum Press, New York, 1987; pp. 109-136). Another type of NFT, predominantly consisting of straight filaments, occurs in progressive supranuclear palsy (synonym: Steele-Richardson-Olszewski syndrome) (Tellez-Nagel I, Wisniewski HM. Ultrastructure of neurofibrillay tangles in Steele-Richardson-Olszewski syndrome. Arch. Neurol. 1973; 29: 324-327). Straight filaments are also the major ultrastructural element of Pick bodies (Wisniewski HM, Coblentz JM, Terry RD. Pick's disease: a clinical and ultrastructural study. Arch. Neurol. 1972; 26:97-108). Pick bodies are round argentophilic neuronal inclusions found in Pick's disease, a less common condition leading to dementia.

It is known that the major protein constituent of the amyloid fibers contained in the senile plaque core and the vascular amyloid deposits is the A4-peptide, an insoluble highly aggregating small polypeptide, also termed beta-amyloid protein (Masters CL, Simms G, Weinman NA, et al. Amyloid plaque protein in Alzheimer's disease and Down's syndrome. Proc. Natl. Acad. Sci. USA 1985; 82:4245-4249 - Glenner GG, Wong CW. Alzheimer's disease: initial report of the purification and characterisation of a novel cerebrovascular amyloid protein. Biochem. Bio-phys. Res. Commun. 1984; 120: 885-890). It is a cleavage product of a larger precursor molecule that exhibits features of a glycosylated cell surface receptor (Kang J. et al., The precursor of Alzheimer's disease amyloid A4 protein resembles a cell-surface receptor. Nature 1987; 325: 733-736). Recently, a serine-protease inhibitor sequence was identified in some forms of this amyloid precursor protein (Tanzi RE, McClatchey AI, Lamperti ED, et al. Protease inhibitor domain encoded by an amyloid protein precursor mRNA associated with Alzheimer's disease. Nature 1988;331:528-530; Ponte P, Gonzales-Dewhitt, Schilling, et al. A new A4 amyloid mRNA contains a domain homologous to serine protease inhibitors. Nature 1988; 334:525-527). The gene coding for the A4 amyloid protein is localised on chromosome 21, at a locus distinct from that of the genetic defect in autosomal dominant Alzheimer's disease (St. George-Hyslop PH et al., The genetic defect causing familial Alzheimer's disease maps on chromosome 21. Science 1987 ; 235 : 885-890 ; Van Broeckhoven C. et al., Failure of familial Alzheimer's disease to segregate with the A4-amyloid gene in several European families. Nature 1987 ; 329: 153-155). In addition, the serine-protease inhibitor $a_1$-antichymotrypsin was recently identified in amyloid deposits of Alzheimer's disease (Abraham CR et al., Immunochemical identification of the serine protease inhibitor a-antichymotrypsin in the brain amyloid deposits of Alzheimer's disease. Cell 1988 ; 52 : 487-501).

In contrast to the amyloid, PHF of Alzheimer's disease have not yet been completely characterised (Perry G., Manetto V, Onorato M, et al. Alterations of the neurofilament-microtubule network in Alzheimer and other neurodegenerative disorders. In: Perry G (Ed) Alterations in the Neuronal Cytoskeleton in Alzheimer's disease. Plenum press, New York, 1987; pp. 137-149). Several lines of evidence, obtained with poly- and monoclonal antibodies, as well as biochemical methods have indicated that neurofilament proteins, proteins associated with microtubules such as MAP-2 and tau, as well as ubiquitin, are associated with PHF. For a more complete disclosure of the mentioned proteins and peptides see more particularly :

- Perry G, Rizzuto N, Autilio-Gambetti L, Gambetti P. Paired helical filaments from Alzheimer's disease patients contain cytoskeletal components. Proc. Natl. Acad. Sci. USA 1985; 82:3916-3920.
- Anderton BH, Breinburg D. Downes MJ, et al, Monoclonal antibodies show that neurofibrillary tangles and neurofilaments share antigenic determinants. Nature 1982; 298: 84-86.
- Miller CJ, Brion JP, Calvert R, et al. Alzheimer's paired helical filaments share epitopes with neurofilament side arms. EMBO J. 1986; 5: 269-276.
- Brion JP, Couck AM, Flament-Durand. J. Neurofibrillary tangles of Alzheimer's disease: an immunohistochemical study. J. Submicrosc Cytol. 1985; 17: 89-96.
- Kosik KS, Duffy LK, Dowling MM, et al. Microtubule-associated protein 2: monoclonal antibodies demonstrate the selective incorporation of certain epitopes into Alzheimer neurofibrillary tangles. Proc. Natl. Acad. Sci. USA 1984; 81: 7941-7945.
- Mori H, Kondo J. Ihara Y. Ubiquitin is a component of paired helical filaments in Alzheimer's disease. Science 1987; 235:1641-1644.
- Perry G, Friedman R, Shaw G. Chau V. Ubiquitin is detected in neurofibrillary tangles and senile plaque neurites of Alzheimer's disease brains. Proc. Natl. Acad. Sci. USA 1987; 84: 3033-3036.

Several of these compounds have also been identified in the 12-15 nm straight filaments comprising Pick bodies (Perry G, Stewart D, Friedman R, et al. Filaments of Pick's bodies contain altered cytoskeletal elements. Amer. J. Pathol. 1987; 127:559-568) and the NFT of progressive supranuclear palsy (Tabaton M, Perry G, Manetto V, et al. Influence of neuronal location on antigenic properties of neurofibrillary tangles. Ann. Neurol. 1988; 23:604-610). However, the prior art of recent literature provides no reliable means for the diagnosis in vitro of neurofibrillar degeneration and a fortiori of Alzheimer's disease.

The present invention provides monoclonal antibodies directed against specific epitopes of proteins involved in various neurofibrillary degeneration, particularly proteins belonging to tangles of Alzheimer's disease.

The invention further provides hybridomas secreting the above said monoclonal antibodies.

The invention provides also antigens involved in abnormal fibrillar structures in degenerating neurones.

The invention also provides a process for the diagnosis in vitro of brain diseases, involving neurofibrillary degeneration, particularly Alzheimer's disease.

A monoclonal antibody of the invention is characterized by the fact that it forms an immunological complex with the syngeneic polyclonal anti-idiotypic serum raised against the monoclonal antibody secreted by the hybridoma deposited at the C.N.C.M. under n°I-883 on July 5, 1989 (NFT200 IgM) and by the hybridoma deposited at the C.N.C.M. under n°I-884 on July 5, 1989 (NFT200 IgG).

A monoclonal antibody of the invention is characterized by the fact that it forms an immunological complex with the monoclonal anti-idiotypic antibody raised against the monoclonal antibody secreted by the hybridoma deposited at the C.N.C.M. under n°I-883 on July 5, 1989 (NFT200 IgM) or by the hybridoma deposited at the C.N.C.M. under n°I-884 on July 5, 1989 (NFT200 IgG).

The monoclonal antibodies of the invention are defined through their idiotype. Idiotypes are sets of idiotopes, i.e. a collection of individually specific antigenic determinants of immunoglobulins (in contrast to allotype and isotype), and can therefore be considered to be a "fingerprint" of an antibody. For full disclosure of idiotype and anti-idiotype, see e.g. de Préval C: Immunoglobulins, p. 144-219 in Bach J.F.: Immunology, Publ. Wiley and Sons, new York, 1978 or Fleischmann J.B., Davie J.M.: Immunoglobulins: allotypes and idiotypes, pp. 205-220 in Paul W.E.:Fundamental Immunology, Publ. Raven Press, New York, 1984. The monoclonal antibodies of the invention specifically react with the anti-idiotypic serum raised against the monoclonal antibody secreted by the hybridoma deposited at the C.N.C.M. under n°I-883 on July 5, 1989 (NFT200 IgM) or by the hybridoma deposited at the C.N.C.M. under n.I-884 on July 5, 1989 (NFT200 IgG), in BALB/c mice in syngeneic conditions. Other BALB/c monoclonal antibodies, of whichever class, subclass or type, fail to react with said syngeneic serum to the monoclonal antibody secreted by the hybridoma deposited at the C.N.C.M. under n°I-883 on July 5, 1989 (NFT200 IgM) and by the hybridoma deposited at the C.N.C.M. under n°I-884 on July 5, 1989 (NFT200 IgG), thus demonstrating the idiotype-specific character of the syngeneic antiserum.

The syngeneic polyclonal anti-idiotypic serum raised against the monoclonal antibody of the invention is obtained by immunization of an animal with a monoclonal antibody of the invention which is raised in a syngeneic, i.e. genetically identical animal, said syngeneic polyclonal serum raised against a monoclonal antibody of the invention containing no other antibodies immunoglobulin than the anti-idiotypic antibodies.

The syngeneic polyclonal anti-idiotypic serum raised against a monoclonal antibody of the invention enables to identify said monoclonal antibody, particularly because said syngeneic polyclonal anti-idiotypic serum does not select for public idiotopes and contains high titers of anti-idiotypic antibodies to private idiotopes of the monoclonal antibodies and particularly because said syngeneic polyclonal anti-idiotypic serum contains and defines the whole set of idiotopes.

The methods used for production of syngeneic anti-idiotype sera to monoclonal antibodies have been described before (Gheuens J., McFarlin D.E., Rammohan K.W., Bellini W.J. : Idiotypes and biological activity of murine monoclonal antibodies against the hemagglutinin of measles virus, Inf. Immun. 34 : 200-207, 1981; Bona C., Hooghe R., Cazenave P.A., Leguern C., Paul W.E. : Cellular basis of regulation of expression of idiotype.II. Immunity to anti-MOPC460 idiotype antibodies increases the level of anti-trinitro-phenyl-antibodies bearing the 460 idiotypes. J. Exp. Med. 149: 815-823, 1979).

The methods for production of monoclonal anti-idiotypic antibodies have been fully described (Gheuens J., McFarlin D.E.: Use of monoclonal anti-idiotypic antibody to P3-X63Ag8 myeloma protein for analysis and purification of B lymphocyte hybridoma products. Eur. J. Immunol. 12: 701-703, 1982).

A monoclonal antibody according to the invention is defined by the combination of the following properties :
- it forms an immunological complex with a non-phosphorylated structural epitope of an antigen released from a sonicated NFT preparation, itself isolated from the cerebral cortex obtained from a patient having Alzheimer's disease - it does not form an immunological complex with any of the following proteins and peptide
- neurofilament proteins
- tau proteins
- MAP-2 protein
- ubiquitin
- 4-amyloid peptide

The expression "the monoclonal antibody of the invention forms an immunological complex with an antigen" means that the monoclonal antibody of the invention binds to the abovesaid antigen under one of the following conditions of the following techniques :

- Light immunomicroscopy :

Brain tissue sample, obtained at surgery or autopsy, is fixed by immersion in 4% formalin or Bouin's fixative and embedded in paraffin. Four mm thick sections are prepared. The monoclonal antibody of the invention is applied either with the avidin-biotinylated peroxidase complex technique (Hsu SM, Raine L, Fanger H. Use of avidin-biotin complex (ABC) in immunoperoxidase techniques. J. Histochem. Cytochem. 1981; 29:577-580), or with the soluble peroxidase-antiperoxidase complex technique (Sternberger LA. Immunocytochemistry (3rd ed.). Wiley, New York, 1986). Diaminobenzidine is used as chromogen.

- Immunoelectron microscopy in tissue sections:

Brain tissue sample, obtained at surgery or autopsy is fixed in either Bouin's fixative or 10% buffered formalin before sectioning 60μm thick without embedding (Vibratome). The sections are immunostained by the indirect immunogold method, fixed, embedded and sectioned for electronmicroscopy as described (Perry G, Mulvihill P, Manetto V, et al. Immunocytochemical properties of Alzheimer straight filaments. J. Neurosci. 1987; 7:3736-3738).

- Immunoelectron microscopy of isolated NFT:

Fractions of enriched PHF, prepared as described (Iqbal K, Zaidi T, Thompson CH, et al., Alzheimer paired helical filaments: bulk isolation, solubility and protein composition. Acta Neuropath. 1984; 62:167-177), are applied to carbon coated nickel grids and immunodecorated by the indirect immunogold method as previously described (Perry G, Mulvihill P, Manetto V, et al. Immunocytochemical properties of Alzheimer straight filaments. J. Neurosci. 1987; 7:3736-3738).

- Immunoblotting procedures :

Fractions enriched in PHF, prepared as described (Iqbal K, Zaidi T, Thompson CH, et al., Alzheimer paired helical filaments: bulk isolation, solubility and protein composition. Acta Neuropath. 1984; 62:167-177), are sonicated and used as samples in SDS-polyacrylamide electrophoresis and immunoblots. SDS-polyacrylamide electrophoresis is done under reducing conditions on 12% gels (Laemmli UK. Cleavage of structural proteins during the assembly of bacteriophage T4. Nature 1970; 227:680-685). After electrophoresis, the proteins are either fixed and stained with Coommassie brilliant blue, or transferred (Towbin H, Staehelin T, Gordon J. Electrophoretic transfer of proteins from polyacrylamide gels to protein binding membranes such as nitrocellulose sheets: procedure and some applications. Proc. Natl. Acad. Sci. USA 1979; 76:4350-4354) to protein binding membranes such as nitrocellulose (Hybond-C, Amersham) or filters commercialized under the name Immobilon (Millipore). After transfer the filters are presoaked in PBS containing 0.05% (v/v) Tween 20 (Tween-PBS) and then incubated for 1 h in Tween-PBS containing 5% (w/v) skimmed dried milk and 5% (v/v) normal goat serum (blocking buffer). Next, the filters are treated overnight at 4°C with primary antibody appropriately in blocking buffer. The filters are then washed three times in Tween-PBS and treated for 1 1/2 h at room temperature with biotinylated goat anti-mouse IgM (Amersham) diluted 1/250 in blocking buffer. After three washes in Tween-PBS, streptavidine-biotinylated horseradish peroxidase complex (Amersham) diluted 1/250 in blocking buffer is applied for 1 1/2 h at room temperature. Afterwards, the filters are washed three times in Tween-PBS and one in PBS. The filters are then incubated in PBS containing 0.05% (w/v) diaminobenzidine and 0.01% (v/v) hydrogen peroxide until background staining developed.

It should be clear that the formation of an immunological complex between the monoclonal antibody and the antigen is not limited to the precised conditions described above, but that all techniques that respect the immunochemical properties of the antibody and antigen binding will produce similar formation of an immunological complex.

The expression "structural epitope" means an epitope defined by the structure, but not the conformation of the antigen. In other words, this epitope is preserved even after treatment of the antigen in ways that will alter its conformation, such as fixation of the antigen with formalin, glutaraldehyde or paraformaldehyde, and after denaturation of the antigen preparation with ionic and non-ionic detergents.

The expression "the monoclonal antibody of the invention does not form an immunological complex with the peptides or proteins" means that a monoclonal antibody of the invention does not bind to above mentioned peptides or proteins in the conditions of light microscopy, electron microscopy and immunoblot technique that are described above.

The "non phosphorylated epitope" means the epitope of the antigen to which the monoclonal antibodies of the invention bind are not phosphorilated, as determined by the following test, which comprises :
- starting from an NFT-enriched fraction prepared as described (Iqbal K, Zaidi T, Thompson CH, et al., Alzheimer paired helical filaments: bulk isolation, solubility and protein composition. Acta Neuropath. 1984; 62:167-177), treated with alkaline phosphatase,
- applying one of the monoclonal antibodies of the invention to said NFT,
- forming an immunological complex between NFT and the monoclonal antibody.

An NFT enriched fraction is immunoblotted to nitrocellulose as described above and treated overnight at 37°C with 1 IU of type III E. coli alkaline-phosphatase in 100 ml 0.1 M Tris buffer, pH 8.0, containing 0.01M phenyl-methyl-sulfonyl-fluoride. Next, monoclonal antibody of the invention is applied, and the immunoblot procedure described above is done.

A monoclonal antibody of the invention is characterized by the fact that it forms an immunological complex with the syngeneic polyclonal anti-idiotypic serum, particularly with the monoclonal anti-idiotypic antibody raised against the monoclonal antibody secreted by the hybridoma deposited at the C.N.C.M. under n°I-883 on July 5, 1989 (NFT200 IgM) and by the hybridoma deposited at the C.N.C.M. under n°I-884 on July 5, 1989 (NFT200 IgG) and by the combination of the following properties :
- it forms an immunological complex with a non-phosphorylated structural epitope of an antigen released from a sonicated NFT preparation, itself isolated from the cerebral cortex obtained from a patient having Alzheimer's disease
- it does not form an immunological complex with any of the following proteins and peptide
- neurofilament proteins
- tau proteins
- MAP-2 protein
- ubiquitin
- 4-amyloid peptide

A particular preferred monoclonal antibody is of the IgM class, kappa type, or of the IgG, kappa type.

Under the experimental conditions described above a preferred monoclonal antibody of the invention does not bind on a non sonicated NFT preparation.

The expression "the monoclonal antibodies do not bind on" means that under the conditions defined above :
- it does not form immunological complexes with any protein or peptide extracted from cortex or white matter extracts of Alzheimer's diseased people or controls with either phosphate buffered solutions (PBS) or sodium dodecyl sulfate containing PBS ;
- it does not form immunological complexes with sonicated NFT preparations previously treated with alkaline phosphatase, obtained as described (Iqbal K, Zaidi T, Thompson CH, et al., Alzheimer paired helical filaments: bulk isolation, solubility and protein composition. Acta Neuropath. 1984; 62:167-177).

The procedure to obtain sonicated NFT preparations previously treated with alkaline phosphatase according to the procedure described in Iqbal et al. will be called in the following of the text "Iqbal procedure".

A preferred monoclonal antibody of the invention was found to form an immunological complex with a non-phosphorylated epitope of an antigen having a molecular weight of about 200.000 d obtained from a sonicated NFT preparation.

Additional characteristics of the monoclonal antibodies of the invention are the following ones :
- they bind immunologically on neurites of seniles plaques,
- they bind immunologically on granulovacuolar degeneration tissue (granulovacuolar degeneration is a specific neuropathological abnormality found in the brain of patients with Alzheimer's disease, see e.g. Price DL, Whitehouse PJ, Struble RG. Cellular pathology in Alzheimer's and Parkinson's diseases. Trends Neurosci. 1986; 9:29-33; Khataturian ZS. Diagnosis of Alzheimer's disease. Arch. Neurol. 1985; 42:1097-1105),
- they bind immunologically on neurofibrillary tangles of supranuclear palsy,
- they bind immunologically on normal axons in peripheral nerves and the spinal cord,
- they bind immunologically on NFT from the brainstem of patients with post-encephalitic Parkinsonism,
- they bind immunologically on neurofibrillary degeneration tissue of Parkinson dementia complex of Guam,
- they bind immunologically on Pick bodies,
- they do not bind immunologically on axons of the cerebral white matter of normal control,
- they do not bind immunologically on Hirano bodies in Alzheimer's disease (Hirano bodies are a specific neuropathological abnormality found in the brain of patients with Alzheimer's disease, see e.g. Price DL, Whitehouse PJ, Struble RG. Cellular pathology in Alzheimer's and Parkinson's diseases. trends Neurosci. 1986; 9:29-33,
- they do not bind immunologically on Lewy bodies in idiophatic Parkinsonism,
- they do not bind immunologically on Rosenthal fibers in cerebellar astrocytoma,
- they do not bind immunologically on normal non-tangle bearing neurons.

The expression "bind immunologically" in the above paragraph refers to the conditions of light and electron microscopy described above.

A preferred monoclonal antibody of the invention is secreted by the hybridoma which has been deposited at the CNCM under n°I-883 on July 5, 1989 (NFT200 IgM).

A preferred monoclonal antibody of the invention is secreted by the hybridoma deposited at the C.N.C.M. under n°I-884 on July 5, 1989 (NFT200 IgG).

The invention also relates to monoclonal antibodies which form immunological complexes with an antigen released from a sonicated NFT preparation, which can be isolated from the cerebral cortex of a patient having Alzheimer's disease, which antigen itself forms an immunological complex with the monoclonal antibody secreted by the hybridoma deposited at the CNCM, under n°I-883 on July 5, 1989 (NFT200 IgM) or by the hybridoma deposited at the C.N.C.M. under n°I-884 on July 5, 1989 (NFT200 IgG).

It is understood that the monoclonal antibodies of the invention do not form immunological complexes with:
- neurofilament proteins,
- tau protein,
- MAP-2 protein,
- ubiquitin,
- 4-amyloid peptide.

The invention also relates to the hybridomas secreting the monoclonal antibodies above defined.

The above mentioned monoclonal antibodies are obtained by a process involving the obtention and isolation of hybridomas secreting the monoclonal antibodies. A process for obtaining the hybridomas comprises - starting from spleen cells of an animal, e.g. mouse or rat, previously immunized in vivo or from spleen cells of such cells previously immunized in vitro with an antigen recognized by the monoclonal antibody secreted by the hybridoma deposited at CNCM under n°I-883 on July 5, 1989 (NFT200 IgM) or by the hybridoma deposited at the C.N.C.M. under n°I-884 on July 5, 1989 (NFT200 IgG),
- fusing said immunized cells with myeloma cells under hybridoma forming conditions and
- selecting those of the hybridomas which secrete the monoclonal antibodies which specifically recognize a non-phosphorylated structural epitope of the abovesaid antigen and which do not immunologically recognize neurofilament proteins, tau proteins, MAP-2 proteins, ubiquitin and 4-amyloid peptides.

A process for producing the corresponding monoclonal antibodies comprises
- culturing the selected hybridomas as indicated above in an appropriate culture medium and
- recovering the monoclonal antibodies excreted by said selected hybridomas,
or alternatively
- implanting the selected hybridomas into the peritoneum of a mouse and, when ascites have been produced in the animal,
- recovering the monoclonal antibodies then formed from said ascites.

The monoclonal antibodies of the invention can be prepared by in vitro conventional techniques such as cultures of immobilized cells using e.g. hollow fibers or microcapsules or such as cultures in homogenous suspension using e.g. airlift reactors.

In vitro immunization of the spleen cells is advantageous because the formation of the monoclonal antibodies is faster. The invention also relates to antigens which form an immunological complex with the monoclonal antibodies of the invention.

The antigen of the invention, which can be obtained from NFT preparations isolated from the cerebral cortex obtained at biopsy or autopsy from a patient with Alzheimer's disease and releasable therefrom by sonication thereof, is characterized by its capability of forming an immunological complex with a monoclonal antibody as defined above, advantageously with the monoclonal antibody secreted by the hybridoma deposited at the CNCM, under n°I-883 on July 5, 1989 (NFT200 IgM) or by the hybridoma deposited at the C.N.C.M. under n°I-884 on July 5, 1989 (NFT200 IgG).

The antigen according to the invention can be obtained by means of a process which comprises
- contacting one of the monoclonal antibodies of the invention with a sonicated preparation of NFT isolated from a patient having had Alzheimer's disease, under conditions suitable for producing an antigen-antibody complex,
- separating the antigen from said complex and recovering the antigen sought in a purified form.

Advantageously, the monoclonal antibodies used are in an immobilized state on a suitable support such as a resin like SEPHAROSE (registered trade mark). The antigen can then be obtained upon
- bringing into contact with said monoclonal antibody the supernatant containing proteins and polypeptides as obtained as a result of an extraction procedure from sonicated NFT preparations with RIPA buffer or a solution with comparable physicochemical properties,
- washing the immobilized antibody-antigen complex then formed,
- treating that complex with any solution, capable of proceeding the dissociation of the antigen-antibody complex (e.g. 3M potassium isothiocyanate, or similar chaotropic reagents) and
- recovering the antigen in a purified form.

RIPA-buffer is 0.01 M Tris-HCl, pH 7.4, 0.15 M NaCl, 1 % sodium desoxycholate, 1 % Triton X100, 0.1 % sodium dodecyl sulfate (SDS), 1 mM phenyl-methyl-sulfonyl-fluoride (PMSF) (=0.5 % of a 200 mM PMSF solution in ethanol) and 500 kappa I.U. aprotinine/ml.

The invention also relates to the syngeneic polyclonal anti-idiotypic serum, and to the monoclonal anti-idiotypic antibodies, raised against the monoclonal antibodies of the invention.

More particularly, the invention relates to the syngeneic polyclonal anti-idiotypic serum, and to the monoclonal anti-idiotypic antibody, raised against the monoclonal antibody secreted by the hybridoma deposited at the C.N.C.M. under n°I-883 on July 5, 1989 (NFT200 IgM) or by the hybridoma deposited at the C.N.C.M. under n°I-884 on July 5, 1989 (NFT200 IgG).

Monoclonal anti-idiotypic antibodies raised against the monoclonal antibodies of the invention can be used to replace the antigen of the invention when they correspond to the internal image of said antigen, for full disclosure on "internal image", see e.g. de Préval C. Immunoglobulins, pp. 144-219 in Bach J.F.; Immunology, Publ. Wiley and Sons, New York, 1978 or Fleischmann J.B., Davie J.M.: Immunoglobulins; allotypes and idiotypes, pp. 205-220 in Paul W.E.; Fundamental Immunology, Publ. Raven Press, New York, 1984; and Jerne, N.K.: Towards a network theory of the immune system Ann. Immunol. (Inst. Pasteur) 125C: 373-389 (1974).

The process of the invention for the detection or diagnosis in vitro of neurofibrillar degeneration, e.g Alzheimer's disease includes:
- contacting in vitro a cell preparation from a patient suspected of suffering of neurofibrillar degeneration, more particularly Alzheimer's disease, with a monoclonal antibody of the invention under conditions suitable for producing an antigen-antibody complex and
- detecting the immunological bonding of said antibody to said cell preparation.

The cell preparation can be produced in any appropriate manner, e.g. from a biopsy obtained from that patient, particularly after sonication thereof and, whenever appropriate, fixing in a suitable medium, e.g. 4% formalin.

The detection of the immunologically bonded monoclonal antibody can be achieved by conventional technology. Advantageously, the monoclonal antibody of the invention carries itself a marker or a group for director indirect coupling with a marker as examplified hereafter.

A particularly advantageous embodiment of the process of the invention comprises contacting a serum preparation or cerebrospinal fluid preparation, obtained from the patient to be diagnosed with the monoclonal antibody of the invention.

The invention also relates to a kit for the diagnosis in vitro of one of the following diseases: Alzheimer's disease, progressive supranuclear palsy, Pick's disease, and all other diseases with pronounced neurofibrillar degeneration, characterized in that it comprises :
at least a suitable solid phase system, e.g. a microtiter-plate for deposition thereon of the biological sample to be diagnosed in vitro ,
- a preparation containing one of the monoclonal antibodies of the invention
- a specific detection system for said monoclonal antibody
- appropriate buffer solutions for carrying out the immunological reaction between a test sample and said monoclonal antibody on the one hand, and the bonded monoclonal antibodies and the detection system on the other hand.

The invention also relates to a kit, as decribed above, also containing a preparation of the antigen of the invention, said antigen of the invention being either a standard (for quantitative determination of the antigen which is sought) or a competitor, with respect to the antigen which is sought, for the kit to be used in a competition dosage process.

The invention also relates to a kit, as decribed above, also containing a preparation of the monoclonal anti-idiotypic antibody of the invention, particularly the one corresponding to the internal image of the antigen of the invention, said monoclonal anti-idiotypic antibody of the invention being either a standard (for quantitative determination of the antigen which is sought) or a competitor with respect to the antigen which is sought, in the case where the kit is used in a competition dosage process.

The invention also relates to corresponding nucleic acids coding for the antigen of the invention, particularly that which is recognized by the antibody secreted by the hybridomas deposited at the C.N.C.M. under n°I-883 on July 5, 1989 (NFT200 IgM) and by the hybridoma deposited at the C.N.C.M. under n°I-884 on July 5, 1989 (NFT200 IgG).

Different methods are of course available to the man skilled in the art to produce or isolate such nucleic acids, particularly (but not necessarily) when the aminoacid sequence of the antigen of the invention becomes available, at least in part, as a result of conventional peptide sequencing procedures.

One of such methods comprises
- contacting under hybridization conditions, a fragmented DNA composition obtained from NFT cells with probes consisting of nucleic acid sequences corresponding to selected amino acid sequences of the antigen of the invention,
- recovering the hybrids formed,
- then separating under suitable denaturing conditions, the DNA strand from the starting probes, said DNA strand then containing at least part of the nucleic acid sequence coding for said antigen.

The process may then further comprises sequencing such strand and after comparison with the possible theoretical nucleotide sequences likely to be in correspondance with the previously determined aminoacid sequences of the antigen of the invention, identifying the relevant part of the nucleic acid.

Another preferred method, more practical and making that nucleic acid more readily accessible, even without preliminary sequencing of at least part of the amino acid sequence of the antigen, comprises:
- recovering the messenger RNAs from cells of the cerebral cortex of a patient having had Alzheimer's disease,
- forming a library cDNAs by reverse transcription of said mRNAs,
- optionally fragmenting said cDNAs, e.g. with a SauIIIA restriction enzyme,
- inserting them in suitable cloning vectors under the control of suitable promoters and regulating elements authorizing the later expression in competent cellular hosts of the open reading frames contained in the inserts then contained in such vectors,
- transforming the appropriate competent cellular hosts with the modified vectors containing such cDNAs or cDNA fragments, and recovering the transformed cell colonies,
- contacting the expression products of said transformed cell colonies, if need be after the lysis of said cells, with a monoclonal antibody according to the invention, under conditions authorizing the production of an immunological complex between said antibody and the corresponding antigen and
- screening these of the cells which produce proteins recognized by the monoclonal antibody of the invention,
- recovering, from the selected cells, the foreign DNA fragment responsible for the expression product which had been recognized by the monoclonal antibody,
- optionally sequencing and identifying that particular nucleic acid and
- finally, after having identified open reading frame which was responsible for the production of the antigen recognized by the monoclonal antibody, recovering a nucleic acid fragment which contained that open reading frame.

Needless to say that this method also provides a method which allows for a determination of the amino acid sequence of at least part of the basic polypeptide structure of the antigen of the invention.

The nucleic acids obtained or fragments thereof can then in turn be used for the production of the antigen of the invention containing sites specifically recognized by the starting monoclonal antibodies, e.g. after insertion of said nucleic acids or fragments in appropriate vectors and transformation of a competent organism with the modified vector to produce the antigen under consideration. Needless to say that under such circumstances, the particular nucleic acid fragment must be inserted again in that vector under the control of a suitable promoter and followed by a suitable termination signal in order to provide for the production of that antigen in the competent host.

The nucleic acid obtained or part thereof can also be used for the production of probes, particularly after radioactive labeling or modification by conventional procedures in order to make them later detectable by suitable markers. In turn, these probes can be used for the detection of the nucleic acid sequences encoding the antigen responsible for Alzheimer's disease or the other diseases referred to in the present application. These probes may either consist of the nucleic acid or part thereof in an isolated form. They may also consist of a recombinant DNA containing said nucleic acid or part thereof, it however being understood that care should then be taken that the other parts of such recombinant DNA probe are not likely to hybridize with the nucleic acid compositions obtained from cell preparations originating from patients to be diagnosed.

Advantageous probes of the invention for detecting the presence of an antigen above defined which comprises a sequence belonging to the nucleic acids above defined or the corresponding complementary nucleic acid, and which includes itself from 10 to the maximum number of nucleotides of said nucleic acids.

Such additional in vitro diagnosis methods then comprise :
- starting from a cell preparation, particularly NFT obtained from the cerebral cortex of a patient suspected of suffering neurofibrillar degeneration, e.g. Alzheimer's disease, whose nucleic acids have been made accessible to possible hybridization with other nucleic acids, whenever required,
- contacting said cell preparation or the nucleic acids previously extracted therefrom with the above defined probe under suitable hybridization conditions, which possibly provides for the production of an hybrid between said probe and a sequence coding for said antigen and
- detecting the hybrid formed, if any, and assessing the possible existence or to the contrary absence of such neurofibrillar degeneration in said patient depending upon the occurence or not of hybridization.

Preferred additional characteristics of the invention will appear in the course of the following description of preferred conditions under which hybridomas and corresponding monoclonal antibodies of the invention can be obtained, which antibodies can then give access to all the other components mentioned above according to procedures which, as such, are all accessible to a person skilled in the art.

EXAMPLE I Preparation of the monoclonal antibody NFT200 (IgM, kappa type)

## ISOLATION OF NFT FOR IMMUNISATION :

NFT were isolated by the long Iqbal procedure (Iqbal K, Zaidi T, Thompson CH, et al. Alzheimer paired helical filaments: bulk isolation, solubility and protein composition. Acta Neuropath (Berl) 1984; 62: 167-177) from the cerebral cortex obtained at autopsy from a patient with apparently sporadic Alzheimer's disease. The diagnosis was neuro pathologically confirmed (Kathaturian ZS. Diagnosis of Alzheimer's disease. Arch. Neurol. 1985; 42:1097-1105). The nature of the NFT preparation was verified by polarized light microscopy after Congo Red staining. This showed flame shaped congophilic NFT, other congophilic material, cell nuclei, capillaries and amorphous debris. The NFT preparation was sonicated before immunisation.

## IN VITRO IMMUNISATION AND FUSION PROCEDURE :

Spleen cells from unimmunised BALB/c mice were cultured in 50% thymocyte conditioned HB 101 medium (Hana Biologicals) with 20% fetal calf serum in the presence of 40 ng/ml sonicated NFT preparation. On the third, fourth and fifth day, $10^8$ spleen cells were mixed with 2 x $10^7$ P3/NS-1/1Ag4-1 myeloma cells and fused in 25% polyethylene glycol for 8 minutes as described (Koprowski H, Gerhard W, Croce CM Production of antibodies against influenza virus by somatic cell hybrids between mouse myeloma and primed spleen cells. Proc. Natl. Acad. Sci. USA 1987; 74: 2985-2988). After each of the three fusions a series of 1440 cultures each containing 5 x $10^4$ cells from the fusion mixture and $10^5$ unimmunised BALB/c spleen cells in 0.2 ml was established in 96-well plates (Nunc) in complete HAT (hypoxanthine aminopterine pyridine) selective medium. This was HB101 medium supplemented with 15% heat-inactivated fetal calf serum and containing hypoxanthine, aminopterine and thymidine. Specific antibody secreting cultures were expanded to 2.5 ml and cloned by limiting dilution.

## SPECIFIC ANTIBODY SCREENING :

a) Materials and methods :

The solid-phase radioimmunoassay to screen hybridoma supernatants for antibody production went as follows. A semi-purified NFT preparation, consisting of the final pellet of the long Iqbal procedure was sonicated and diluted to approximately 10 $\mu$g protein/ml in 0.1 M sodium bicarbonate. This antigen was adsorbed to polyvinyl chloride flat-bottom 96-well plates (Falcon) as described. Fifty $\mu$l of hybridoma supernatant was applied to each well. After 4 h at 4° C, the wells were washed three times with 0.1 M sodium phosphate buffer (pH 7.4) containing 0.15 M sodium chloride (PBS) and 2% fetal calf serum, and incubated with 50 $\mu$l I$^{125}$ labeled anti-mouse immunoglobulin (25.000 cpm) (Amersham). After 2h, the wells were washed three times, cut from the plate with a hot wire and counted for 1 mn in a gamma counter. A sample was considered positive when the cpm bound by it were greater than the mean plus two standard deviations of the cpm bound by complete HAT medium.

b) Result s :

Hybridoma cell growth occured in 829 of the 4320 cultures. Twenty three stable antibody secreting hybridomas were identified with the screening assay. The yield of specific hybridomas was the same for each fusion, irrespective of the duration of in vitro antigen priming. One of these monoclonal antibodies, termed NFT 200, is described further. It was unambiguously of the IgM class, kappa type.

LIGHT IMMUNOMICROSCOPY :

a) Material and methods :

Brain tissue of apparently sporadic presents (N = 4) and senile (N = 4) Alzheimer's disease, autosomal dominantly inherited Alzheimer's disease in two large Belgian families (N = 14) (Van Broeckhoven et al, 1987, Failure of familial Alzheimer disease to segregate with the A4-amyloid gene in several European families. Nature 329, 153-155), Parkinson dementia complex (N = 3) and amyotrophic lateral sclerosis (N = 2) of Guam, a person from Guam with NFT but without neurological disease, Pick's disease (N = 3), post-encephalitic (N = 1) and idiopathic (N = 1) Parkinsonism, progressive supranuclear paralysis (N = 3), subacute sclerosing panencephalitis (N = 1), cerebellar astrocytoma with Rosenthal fibers (N = 1), ganglioma (N = 2), sudden infant death syndrome (N = 2) was taken at autopsy. Cerebral cortex of patients obtained at surgery for brain tumor (N = 5) and normal age-matched controls (N = 3) were used as controls. Control human spinal cord and peripheral nerve, rat sciatic nerve and other human tissues (adrenal, lung, liver, skeletal muscle, kidney, ovary, pancreas, spleen) were also studied. Each tissue sample was fixed by immersion in 4% formalin and embedded in paraffin. Four mm thick sections were prepared.

The monoclonal antibody of the invention was a applied either with the avidin biotinylated peroxidase complex technique (Hsu SM, Raine L, Fanger H. Use of avidin-biotin complex (ABC) in immunoperoxidase techniques. J. Histochem. Cytochem. 1981; 29: 577-580), or with soluble peroxidase-anti-peroxidase complex technique (Sternberger LA. Immunocytochemistry (3rd.) Wiley, New York, 1986).

Diaminobenzidine was used as chromogen. Monoclonal anti-glial fibrillary acidic protein (anti GFAP)(Gheuens J, De Schutter E, Noppe M, Lowenthal A. Identification of several forms of the glial fibrillary acidic protein, or a-albumin, by a specific monoclonal antibody. J. Neurochem. 1984; 43: 964-970) and the myeloma proteins MOPC21 (IgG1) and MOPC1O4E (IgM) (Litton Bionetics) served as controls. In addition, polyclonal rabbit antisera to GFAP, paired helical filaments and neuro-filaments were studied with the soluble peroxidase-anti-peroxidase complex technique. Here, preimmune rabbit serum served as control. Conventional cresyl violet, Holzer, Spielmeyer, Bodian, Von Braunmuhl, PAS and Congo Red stainings were also done in each case. The Congo Red sections were examined in polarised light.

b) Results :

In presenile and senile Alzheimer's disease, of either sporadic or familial nature, NFT200 intensely labelled NFT in perikarya in different region of the cerebrum and the brain stem. Most of these NFT were congophilic and showed green birefringency. Neurites in senile plaques and at some distance from plaques and perikarya were also labeled, as well as granulovacuolar degeneration. In all instances, the amyloid containing senile plaque core and the vessel walls remained negative.

NFT200 also labeled NFT in the brainstem in patients with post-encephalitic Parkinsonism. In this instance NFT were frequently of the "globoid" type. Here, NFT were almost exclusively localised in the perikaryon. Only rarely, were neurites stained at a distance from the cell soma. In a cerebral cortical biopsy and subsequent autopsy of a patient with subacute sclerosing panencephalitis NFT 200 demonstrated NFT in a few pyramidal neurons. These flame-shaped NFT could not be distinguished from those occuring in Alzheimer's disease. In this patient neurites were not stained. The neurofibrillary degeneration of Parkinson dementia complex of Guam was also intensely labeled.

The NFT of progressive supranuclear paralysis were also labelled with NFT200. In Pick's disease, Pick bodies were numerous in cortical layers II and IV, and intensely labelled with NFT200. The Pick bodies showed arounded contour and irregularly arranged fibrils. In ballooned neurons with central chromatolysis, small NFT200 immunoreactive granules were found at the peripheral rim of the perikaryon.

In normal controls , NFT200 labeled the axons in peripheral nerves and in the spinal cord, but not in the cerebral white matter. Occasionally, a weak labelling of axons in the optic nerve was observed. Dendrites remained negative, as did the brain tissue of infants. Sporadically occuring NFT in age matched controls were also stained. All other tissues examined were negative. NFT200 also labelled the axons in rat sciatic nerve.

NFT200 did not immunostain Hirano bodies in AD, nor Lewy bodies in idiopathic Parkinsonism, while the periphery of Lewy bodies was labelled with polyclonal anti-neurofilament serum. Rosenthal fibers in cerebellar astrocytoma also remained negative.

IMMUNOELECTRON MICROSCOPY IN TISSUE SECTIONS :

a) Material and methods :

Hippocampal tissue from sporadic and familial cases of Alzheimer's disease, frontal and temporal cortex from a Pick case, the brain stem from a case of progressive supranuclear paralysis, and peripheral nerve were fixed in either Bouin's fixative or 10% buffered formalin before sectioning 60 μm thick without embedding (Vibratome). The sections were immunostained by the indirect immunogold method, fixed, embedded and sectioned for electronmicroscopy as previously described (Perry G., Mulvihill P, Manetto V, et al. Immunocytochemical properties of Alzheimer straight filaments. J. Neurosci. 1987; 7:3736-3738).

b) Results :

Ultrastructurally, NFT200 tagged paired helical filaments and granulovacuolar degeneration in Alzheimer's disease. Amyloid filaments, or amyloid deposits in senile plaque core and vessel walls were not labeled. In Pick's disease and in progressive supranuclear paralysis, the 12-15 nm straight filaments were labeled. In peripheral nerve specific cytoplasmic staining of axons, not limited to cytoskeletal structures was observed with NFT200.

IMMUNOELECTRON MICROSCOPY OF ISOLATED NFT :

a) Material and methods :

Fractions of enriched PHF, prepared as the immunogen used for immunisation before sonication, were applied to carbon coated nickel grids and immunodecorated by the indirect immunogold method as previously described under the paragraph titled "immunoelectron microscopy in tissue section" (Perry G., Mulvihill P, Manetto V, et al. Immunocytochemical properties of Alzheimer straight filaments. J. Neurosci. 1987; 7:3736-3738).

b) Results :

The reactivity of NFT200 with PHF in situ was confirmed on immunonegatively stained isolated PHF from Alzheimer's disease.

IMMUNOBLOTTING PROCEDURES :

a) Materiel and methods :

Fractions enriched in PHF, prepared as the immunogen used for immunisation were sonicated and used as samples in SDS-polyacrylamide electrophoresis and immunoblots. In addition, PBS and SDS extracts of the cerebral cortex of a case of sporadic Alzheimer's disease, a case of familial AD and a case of adrenoleukodystrophy were prepared as described (Gheuens J, see above) and used as samples. Similar extracts were prepared from the hemispherical white matter of the same cases. Neurofilament protein purified from control human spinal cord (Koon-Sea Hui, Maria Hui, Fung-Chow Chiu, Miriam Banay-Schawartz, Teresita Deguzman, Robert S. Sacchi, Abel lajtha: Seperation and purification of individual neurofilament proteins by reverse-phase high-performance liquid chromatography. Anal. Biochem. 153: 230:234 (1986), heat-stable microtubule associated proteins (kind gift from J. Avilla), ubiquitin (Sigma) and synthetic A4-amyloid peptide (kind gift from K. Beyreuther) were also analysed.

SDS-polyacrylamide electrophoresis was done under reducing conditions on 12% gels (Laemmli UK. Cleavage of structural proteins during the assembly of bacteriophage T4. Nature 1970; 227: 680-685). After electrophoresis, the proteins were either fixed and stained with Coommassie Brilliant Blue, or transferred (Towbin H, Staehelin T, Gordon J. Electrophoretic transfer of proteins from polyacrylamide gels to nitrocellulose sheets: procedure and some applications. Proc. Natl. Aca. Sci. USA 1979; 76: 4350-4354) to nitrocellulose (Hybond-C, Amersham) or PVD FILTERS (Immobilon, Millipore). After transfer the filters were presoaked in PBS containing 0.05% (v/v) Tween 20 (Tween-PBS) and then incubated for 1 h. in Tween-PBS containing 5% (w/v) skimmed dried milk and 5% (v/v) normal goat serum (blocking buffer). Next, the filters were treated overnight at 4°C with primary antibody appropriately diluted in blocking buffer. The filters were then washed three times in Tween-PBS and treated for 1 1/2 h at room temperature with biotinylated goat anti-mouse IgM (Amersham) diluted 1/250 in blocking buffer. After three washes in Tween-PBS, streptavidine-biotinylated horseradish peroxidase complex (Amersham) diluted 1/250 in blocking buffer was applied for 1 1/2 h at room temperature. Afterwards, the filters were washed three times in Tween-PBS and once in PBS. The filters were then incubated in PBS containing 0.05% (w/v) diaminobenzidine and 0.01% (v/v) hydrogen peroxide until background staining developed. MOPC21 and MOPC104E myeloma proteins were used as negative controls. Anti-neurofilament monoclonal antibody, antitau, anti-MAP2 and anti-amyloid antibodies were used as controls in immunoblots of the corresponding antigens.

b) Result s :

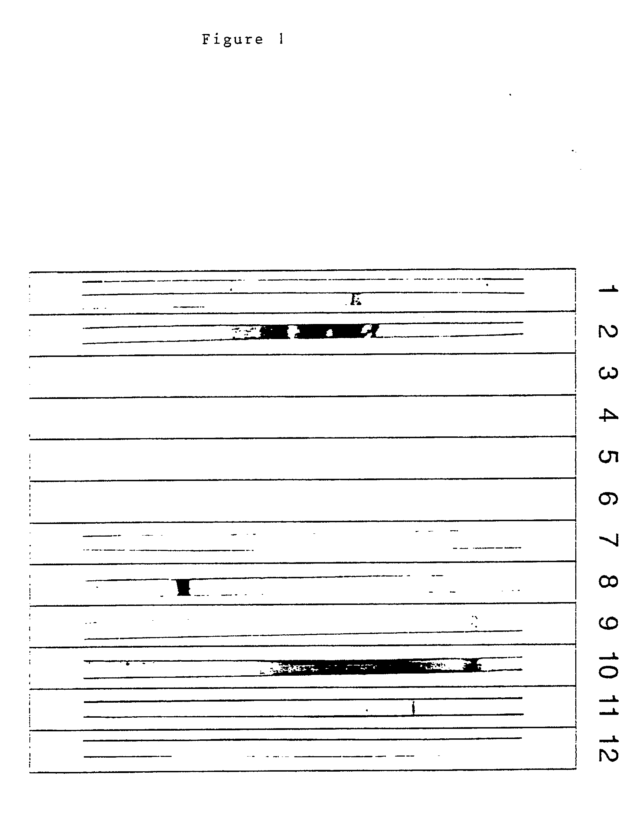

NFT200 did not cross-react with isolated neurofilament proteins, tau protein, MAP2 protein, ubiquitin or the A4-amyloid peptide. The antibody did not react with any peptide in the aqueous and SDS extracts of cortex or white matter extracts from either AD or control. However, immunoblots with sonicated PHF enriched fractions indicated that the NFT200 antibody recognised a 200KD protein band (Fig. 1). More precisely, Fig. 1 shows that NFT 200 does not react in immunoblots with neurofilament proteins (lane 1) (lane 2: monoclonal anti-neurofilament antibody), tau protein (lane 3) (lane 4 : anti-tau antibody), MAP-2 (lane 5) (lane 6: anti-MAP-2 antibody), ubiquitin (lane 7) (lane 8: anti-ubiquitin antibody), or A4-amyloid peptide (lane 9) (lane 10 : anti-A4 antibody). NFT 200 recognizes a protein band with a molecular weight of 200,000 in sonicated PHF enriched fractions (lane 11), but not in aqueous extracts from cortex of an Alzheimer's disease patient (lane 12).

DEPHOSPHORYLATION EXPERIMENTS :

a) Material and methods :

To assess if the antibody of the invention was directed against a phosphorylated epitope semi-purified NFT immunoblotted to nitrocellulose as described above were treated overnight at 37°C with 1 IU of Type III E.coli alkaline-phosphatase in 100 µl 0.1 M Tris buffer, pH 8.0, containing 0.01 M phenyl-methyl-sulfonyl-fluoride. Next, the monoclonal antibody of the invention was applied, and the immunoblot procedure described above was done. GFAP and monoclonal anti-GFAP, as well as neurofilament proteins and monoclonal anti-neurofilament antibody served as controls. The monoclonal anti-GFAP was directed against a non-phosphorylated epitope, the monoclonal anti-neurofilament antibody was directed against a phosphorylated epitope (Gheuens et al. unpublished results).

b) Results :

Semi-purified NFT that were treated with alkaline-phosphatase retained their reactivity with NFT200. This indicated that NFT200 was not directed against a phosphorylated epitope.

OTHER METHODS :

Class and type determination of the monoclonal antibody was done with class and type-specific anti-mouse immunoglobulin reagents (Litton Bionetics) in Ouchterlony double immunodiffusion experiments. Sephacryl S300 (Pharmacia) gel filtration chromatography was used to purify the NFT200 monoclonal antibody from hybridoma ascites.

EXAMPLE II : Preparation of a switch variant monoclonal antibody (of the IgG, kappa type) from the NFT200 hybridoma culture.

1) Selection of an IgG₁, kappa type secreting switch variant from the NFT200 hybridoma culture:

The NFT200 hybridoma secretes monoclonal antibody of the IgM class, kappa type. However, IgM-monoclonal antibodies can offer technical disadvantages over IgG-monoclonal antibodies in immunochemical application. We have therefore devised a method by which it was possible to select and clone a variant that secretes $IgG_1$, kappa with identical antigenic specificity as the IgM, kappa NFT200 monoclonal antibody from an NFT200 hybridoma culture. Such so-called "switch-variants", that secrete antibody of a different class, are known to occur spontaneously in IgM-secreting hybridomas. However, such events are rare, and estimated to occur at a frequency of only $10^{-5}$ to $10^{-8}$. The desired switch-variant therefore has to be identified, selected and cloned from the IgM-producing hybridoma quickly, before overgrowth occurs.

The methods that was used went as follows. A series of 480 cultures, each containing $10^3$ NFT200 hybridoma cells in 0.15 ml complete medium was established in five 96-well plates. Complete medium was Dulbecco's Modified Eagle Medium, with high glucose (4.500 mg/L), supplemented with 1.2 mM sodium pyruvate, 2 mM glutamine, 100 I.U./ml penicilline, 100 I.U./ml streptomycine and 10 % heat-inactivated (30 minutes at 56° C) fetal calf serum. After incubation for 5 days at 37° C in a 5 % $CO_2$ atmosphere, each culture was screened for $IgG_1$ production (as described hereafter). Positive cultures were plated out at 200 cells in 0.15 ml complete medium in 96-well plates, and screened for $IgG_1$ production after 5 days. The cultures that secreted $IgG_1$ were then cloned in three series of 0.1 ml cultures, respectively containing 100, 10 and 0.5 cells per well, and each containing $10^5$ BALB/c thymocyte feeder cells. After 6 days the wells in which cell growth had occurred were screened for the presence of $IgG_1$ production and the absence of IgM production. The $IgG_1$ positive, IgM negative cultures were then recloned as described above at 0.3 cells/well, and tested again after 7 days. An $IgG_1$ secreting switch-variant of the NFT200 hybridoma was thus isolated. The antibody was tested for antigen specificity as described for the NFT200 monoclonal antibody. The results indicated that the $IgG_1$ switch-variant monoclonal antibody had the same antigen-specificity as the IgM NFT200. It was also demonstrated that the $IgG_1$ switch-variant monoclonal antibody expressed the same idiotype as the NFT2OO IgM monoclonal antibody (as described hereafter).

**2) Screening assay for detection of $IgG_1$ secretion by hybridoma cultures**

Polyvinyl chloride flat-bottom 96-well plates (Nunc F16 Maxisorp) were coated overnight at 4° C with 100 $\mu$l polyclonal rabbit anti-mouse $IgG_1$ serum (Organon), diluted 1:1000 in 0.01 M sodium chloride -0.01 M sodium azide - 0.01 M Tris buffer, pH 8.5. All subsequent incubations were at 37° C.

After two washes with PBS containing 0.05 % Tween 80 (Tween-PBS), the plates were saturated for 1 h with 150 $\mu$l 10 % casein in PBS (casein-PBS). Next the plates were washed three times with Tween-PBS, and 100 $\mu$l of the supernatant to be tested was added. After 1 h, the plates were washed again, and 100 $\mu$l of biotinylated goat anti-mouse $IgG_1$ serum (Amersham), diluted 1:2000 in casein-PBS was added to each well. After 1 h, the plates were washed three times, and incubated with 100 $\mu$l streptavidine-biotinylated peroxidase complex (Amersham RPN 1051), diluted 1:1000 in casein-PBS for 30 minutes. Next, the plates were washed four times with Tween-PBS, and 100 $\mu$l 0.0004 M 3,3',5,5'-tetramethylbenzidine (TMB) (Boehringer 784974) in 0.1 M phosphate-citrate buffer, pH 4.3, containing 0.004 % hydrogen peroxide was added to each well. After 30 minutes at 37° C, the reaction was stopped by adding 50 $\mu$l 2 M sulfuric acid to each well. Absorption was then read at 450 nm.

To detect IgM secretion by the hybridomas, the plates were coated with polyclonal rabbit anti-mouse IgM serum and biotinylated goat anti-mouse IgM serum was used as second reagent. Otherwise, the screening assay was identical to that described above.

EXAMPLE III: Preparation of syngeneic monoclonal anti-idiotypic antibodies to NFT200:

The NFT200 IgM, kappa monoclonal antibody, as well the $IgG_1$ kappa monoclonal antibody that is secreted by an $IgG_1$, kappa switch variant of the NFT200 hybridoma (see preceding paragraph) are defined through their idiotype, as described earlier

1) Production of syngeneic anti-idiotype sera to the monoclonal antibody NFT200.

NFT200 monoclonal antibody was purified from NFT200 hybridoma ascites by gel filtration FPLC over Sephacryl S-300 (column C26,L:86 cm). The sample was 10 ml NFT200 hybridoma ascites, diluted 1:2 in 0.08 M phosphate buffer with 0.077 M sodium chloride. The flow rate was 10 ml/h. The NFT200 monoclonal antibody appeared in the void volume. The purified NFT200 was then concentrated to 1 mg/ml in saline. BALB/c mice, 10 to 12 weeks of age, were immunised as follows. Purified NFT200 was conjugated to keyhole limpet hemocyanin (KLH) (Calbiochem) (1 mg/ml in saline) with glutaraldehyde as described in the paper by Bona et al. The resulting NFT200-KLH conjugate was emulsified in complete Freund adjuvant to give 75 $\mu$g of NFT200-KLH conjugate and 100 $\mu$g of Mycobacterium tuberculosis H37 Ra (Difco) in 100 $\mu$l of emulsion. Each mouse was given 50 $\mu$l of this emulsion intraperitoneally, and another 50 $\mu$l was distributed among the hind legs footpads and the inguinal and axillary regions. Five days after the first immunisation, 75 $\mu$g of NFT200-KLH conjugate

in 100 μl of incomplete Freund adjuvant was injected over the axillary and inguinal regions. The mice were boosted once weekly with 75 μg of conjugate in saline given in the axillary and inguinal regions. The mice were first bled 6 days after injection 6 and subsequently every 2 weeks. The anti-idiotypic nature of the syngeneic antisera is correlated to immunological specificity of the antiserum to NFT200, and products derived from it, but not to other BALB/c immunoglobulins of the IgM class, kappa type, nor any other BALB/c immunoglobulin. This is done using appropriate ELISA procedures, or agglutination assays with immunoglobulin-coated sheep red blood cells, as described in the papers under reference.

**2) Production of syngeneic monoclonal anti-idiotypic antibodies to NFT200**

Spleen cells, taken from a mouse that has been immunised with NFT200-KLH conjugate as described in the preceding paragraph, can be fused to BALB/c myeloma cells in the manner described elsewhere in this application, to produce hybridoma cell cultures. The hybridoma cultures can be screened for the production of antibodies to NFT200 monoclonal antibody using the multivalent antibody radioimmunoassay, that has been described before (Gheuens J., McFarlin D.E.: Multivalent antibody radioimmunoassay (MARIA) for screening specific antibody secretion by lymphocyte hybridoma cultures. Meth. Enzymol. 121: 425-433, 1986). The anti-idiotypic nature of such monoclonal antibodies to NFT200 could be assessed as described in the papers under reference. The anti-idiotypic nature of antibodies is correlated to the immunological specificity of such antibodies to NFT200, and products derived from it, but not to other BALB/c immunoglobulins of the IgM class, kappa type, nor any other BALB/c immunoglobulin.

**Claims**

1. Monoclonal antibody characterized by the combination of the following properties :
- it forms an immunological complex with a non-phosphorylated structural epitope of an antigen released from a sonicated NFT preparation, which itself is isolated from the cerebral cortex obtained from a patient having Alzheimer's disease,
- it does not form an immunologically complex with the following proteins or peptides :
. neurofilament proteins
. tau protein
. MAP 2 protein
. ubiquitin

. 4-amyloid peptide
2. Monoclonal antibody characterized by the fact that it forms an immunological complex with the syngeneic polyclonal anti-idiotypic serum, particularly with the monoclonal anti-idiotypic antibody, raised against the monoclonal antibody secreted by the hybridoma deposited at the C.N.C.M. under n°I-883 on July 5, 1989 (NFT200 IgM) or by the hybridoma deposited at the C.N.C.M. under n°I-884 on July 5, 1989 (NFT200 IgG).
3. Monoclonal antibody characterized by the combination of the following properties :
- it forms an immunological complex with a non-phosphorylated structural epitope of an antigen released from a sonicated NFT preparation, which itself is isolated from the cerebral cortex obtained from a patient having Alzheimer's disease,
- it does not form an immunologically complex with the following proteins or peptides :
. neurofilament proteins
. tau protein
. MAP 2 protein
. ubiquitin
. 4-amyloid peptide
and characterized by the fact that it forms an immunological complex with the syngeneic polyclonal anti-idiotypic serum, particularly with the monoclonal anti-idiotypic antibody, raised against the monoclonal antibody secreted by the hybridoma deposited at the C.N.C.M. under n°I-883 on July 5, 1989 (NFT200 IgM) and by the hybridoma deposited at the C.N.C.M. under n°I-884 on July 5, 1989 (NFT200 IgG).
4. Monoclonal antibody according to claims 1 to 3, which is of the IgM class, kappa type or of the IgG, kappa type.
5. Monoclonal antibody according to anyone of claims 1 to 4, which does not bind on a non sonicated NFT preparation.
6. Monoclonal antibody according to anyone of claims 1 to 5, which :
- does not form an immunological complex with any protein or peptide extracted from cortex or white matter extracts of Alzheimer's diseased people or controls, with either water or sodium dodecyl sulfate solutions or phosphate buffered solutions,
- does not form an immunological complex with sonicated NFT preparations previously treated with alkaline phosphatase, obtained according to Iqbal procedure.
7. Monoclonal antibody according to anyone of claims 1 to 6, which forms an immunological complex with a non-phosphorylated epitope of an antigen of molecular weight of about 200 kd, obtained from a sonicated NFT preparation.
8. Monoclonal antibody according to anyone of claims 1 to 7, which :
- binds immunologically on neurites in seniles plaques,
- binds immunologically on granulovacuolar degeneration,
- binds immunologically on the neurofibrillary tangles of supranuclear palsy,
- binds immunologically on normal axons in peripheral nerves and the spinal cord,
- binds immunologically on the NFT from the brainstem of patients with post-encephalitic Parkinsonism,
- binds immunologically on the neurofibrillary degeneration tissue of Parkinson dementia complex of Guam,
- binds immunologically on Pick bodies,
- does not bind immunologically on axons of the cerebral white matter of normal control,
- does not bind immunologically on Hirano bodies in Alzheimer's disease,
does not bind immunologically on Lewy bodies in idiophatic Parkinsonism,
- does not bind immunologically on Rosenthal fibers in cerebellar astrocytoma.

9. Monoclonal antibody secreted by the hybridoma which has been deposited at the CNCM, under n°I-883 on July 5, 1989 (NFT200 IgM) and by the hybridoma deposited at the C.N.C.M. under n°I-884 on July 5, 1989 (NFT200 IgG).

10. Monoclonal antibody which forms an immunological complex with an antigen released from a sonicated NFT preparation isolated from the cerebral cortex of a patient having Alzheimer's disease, which antigen itself forms an immunologically complex with the monoclonal antibody secreted by the hybridoma deposited at the CNCM, under n°I-883 on July 5, 1989 (NFT200 IgM) and by the hybridoma deposited at the C.N.C.M. under n°I-884 On July 5, 1989 (NFT200IgG).

11. Monoclonal antibody according to claim 10 which does not form an immunological complex with the
. neurofilament proteins
. tau protein
. MAP-2 protein
. ubiquitin
. 4-amyloid peptide

12. Hybridoma which secretes the monoclonal antibody of anyone of claims 1 to 11.

13. Antigen releasable by a sonicated NFT preparation, isolated from the cerebral cortex from a patient having Alzheimer's disease and which forms an immunological complex with a monoclonal antibody of anyone of claims 1 to 11.

14. Antigen which forms an immunological complex with the monoclonal antibody secreted by the hybridoma deposited at the CNCM, under n°I-883 on July 5, 1989 (NFT200 IgM) or by the hybridoma deposited at the C.N.C.M. under n°I-884 on July 5, 1989 (NFT200 IgG).

15. Monoclonal anti-idiotypic antibody characterized by the fact that it forms an immunological complex with anyone of the monoclonal antibodies of claims 1 to 11.

16. Monoclonal anti-idiotypic antibody characterized by the fact that it forms an immunological complex with the monoclonal antibody secreted by the hybridoma deposited at the C.N.C.M. under n°I-883 on July 5, 1989 (NFT200 IgM) and by the hybridoma deposited at the C.N.C.M. under n°I-884 on July 5, 1989 (NFT200 IgG).

17. Process for obtaining and isolating hybridomas secreting a monoclonal antibody according to anyone of claims 1 to 11 which comprises
- starting from spleen cells of an animal, e.g. mouse or rat, previously immunized in vivo or from spleen cells of such cells previously immunized in vitro with an antigen recognized by the monoclonal antibody deposited at CNCM under n°I-883 on July 5, 1989 (NFT200 IgM) or by the hybridoma deposited at the C.N.C.M. under n°I-884 on July 5, 1989 (NFT200 IgG), - fusing said immunized cells with myeloma cells under hybridoma forming conditions and
- selecting those of the hybridomas which secrete the monoclonal antibodies which specifically recognize a non-phosphorylated structural epitope of the antigen according to claim 13 or 14 and which do not immunologically recognize neurofilament proteins, tau proteins, MAP-2 proteins, ubiquitin and 4-amyloid peptide.

18. Process for producing monoclonal antibodies according to claims 1 to 11, which comprises
- culturing the selected hybridomas according to claim 12, in an appropriate medium culture and
- recovering the monoclonal antibodies excreted by said selected hybridomas or alternatively
- implanting the selected hybridomas into the peritoneum of a mouse and when ascites have been produced into the animal recovering the monoclonal antibodies then formed from said ascites .

19. Process for the preparation of the antigen according to claim 13 or 14, which comprises - starting from nucleic acids or fragments thereof coding for the antigen according to claims 11 or 12 and inserting said nucleic acids or fragments in an appropriate vector, under the control of a suitable promoter and followed by a suitable termination signal,
- transforming a competent organism with the modified vector to produce said antigen.

20. Process for the detection or diagnosis in vitro of the neurofibrillar degeneration, e.g. in Alzheimer's disease, progressive supranuclear palsy, Pick's disease, and all other diseases with pronounced neurofibrillar degeneration which includes :
- contacting in vitro a cell preparation from a patient suspected of suffering of neurofibrillar degeneration, more particularly Alzheimer's disease, with a monoclonal antibody according to anyone of claims 1 to 11, under conditions suitable for producing an antigen-antibody complex and
- detecting the immunological bonding of said antibody to said cell preparations.

21. Kit for the diagnosis **in vitro,** of one of the following disease: Alzheimer's disease, progressive supranuclear palsy, Pick's disease, and all other diseases with pronounced neurofibrillar degeneration which comprises :
- at least a suitable solid-phase system, e.g. a microtiter-plate for deposition thereon of the biological sample to be diagnosed **in vitro,** - a preparation containing a monoclonal antibody of anyone of claims 1 to 11,
- a specific detection system for said monoclonal antibody,
- appropriate buffer solutions for carrying out the immunological reaction between a test sample and said monoclonal antibody on the one hand, and the bonded monoclonal antibodies and the detection system on the other hand,
- possibly the antigen according to claim 13 or 14 or the monoclonal anti-idiotypic antibody according to claims 15 or 16, for standard purposes or for competition purposes with respect to the antigen which is sought.

22. Nucleic acid sequence coding for the antigen according to claim 13 or 14.

23. Probe for detecting the presence of the antigen according to claim 13 or 14, which comprises :
- a sequence belonging to the nucleic acid of claim 22, or to the complementary nucleic acid of claim 22, and which includes itself from 10 to the maximum number of nucleotides of the nucleic acid of claim 22.

24. Process for the detection or diagnosis **in vitro** of neurofibrillar degeneration, such as Alzheimer's disease which comprises
- starting from a cell preparation, particularly NFT obtained from the cerebral cortex of a patient suspected of suffering neurofibrillar degeneration, e.g. Alzheimer's disease, whose nucleic acids have been made accessible to possible hybridization with other nucleic acids, whenever required,
- contacting said cell preparation or the nucleic acids previously extracted therefrom with the above defined probe under suitable hybridization conditions possibly provide for the production of an hybrid between said probe and a sequence coding for said antigen and
- detecting the hybrid formed, if any, and assessing the possible existence or to the contrary absence of such neurofibrillar degeneration in said patient depending upon the occurence or not of hybridization.

Figure 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-8 903 993  (MEDICAL RESEARCH COUNCIL)<br>* The whole document *<br>– – – | 1-14,<br>17-24 | C 12 P 21/08<br>G 01 N 33/577 //<br>C 12 N 15/12 |
| Y | | 15,16 | C 12 Q 1/68 |
| X | BIOLOGICAL ABSTRACTS, vol. 78, abstract no. 21313, Biological Abstracts, Inc., Philadelphia, PA, US; G.P. WANG et al.: "Alzheimer neuro fibrillary tangles mono clonal antibodies to inherent antigens",<br>& ACTA NEUROPATHOL 62(4), 1984, 268-275<br>– – – | 1-14,17,<br>18 | |
| X | AM. J. PATHOL., vol. 126, no. 1, January 1987, pages 81-91; S.-H. YEN et al.: "Alzheimer's neurofibrillary tangles contain unique epitopes and epitopes in common with the heat-stable microtubule associated proteins tau and MAP2"<br>* The whole document *<br>– – – | 1-14,17,<br>18 | |
| X | BRAIN RESEARCH, vol. 372, 1986, pages 80-88, Elsevier Science Publishers B.V. (Biomedical Division); R. RUBENSTEIN et al.: "Paired helical filaments associated with Alzheimer disease are readily soluble structures"<br>* The whole document *<br>– – – | 1-14,17,<br>18 | |
| D,Y | EUR. J. IMMUNOL., vol. 12, 1982, pages 701-703; J. GHEUENS et al.: "Use of monoclonal anti-idiotypic antibody to P3-X63Ag8 myeloma protein for analysis and purification of B lymphocyte hybridoma products"<br>* The whole document *<br>– – – | 15,16 | **TECHNICAL FIELDS SEARCHED (Int. Cl.5)**<br><br>C 12 P |
| A | ANN. NEUROL., vol. 25, no. 2, 1989, pages 145-151; S.K. SHANKAR et al.: "Immunocytochemical characterization of neurofibrillary tangles in amyotrophic lateral sclerosis and parkinsonism-dementia of guam"<br>– – –<br><br>–/– | | |

The present search report has been drawn up for all claims

| Place of search | Date of completion of search | Examiner |
|---|---|---|
| The Hague | 29 October 90 | SKELLY J.M. |

European
Patent Office

**EUROPEAN SEARCH
REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | ACTA NEUROPATHOL., vol. 62, 1984, pages 167-177; K. IQBAL et al.: "Alzheimer paired helical filaments: Bulk isolation, solubility, and protein composition"<br>- - - - - | | |

TECHNICAL FIELDS
SEARCHED (Int. Cl.5)

The present search report has been drawn up for all claims

| Place of search | Date of completion of search | Examiner |
|---|---|---|
| The Hague | 29 October 90 | SKELLY J.M. |

CATEGORY OF CITED DOCUMENTS

X : particularly relevant if taken alone
Y : particularly relevant if combined with another
document of the same catagory
A : technological background
O : non-written disclosure
P : intermediate document
T : theory or principle underlying the invention

E : earlier patent document, but published on, or after
the filing date
D : document cited in the application
L : document cited for other reasons

&: member of the same patent family, corresponding
document